(19) Europäisches Patentamt
European Patent Office
Office européen des brevets

(11) Numéro de publication : **0 661 278 A1**

# (12) DEMANDE DE BREVET EUROPEEN

(21) Numéro de dépôt : **94402966.9**

(22) Date de dépôt : **21.12.94**

(51) Int. Cl.[6] : **C07D 251/34,** C07D 229/00, C08G 18/80

(30) Priorité : **31.12.93 FR 9316036**

(43) Date de publication de la demande : **05.07.95 Bulletin 95/27**

(84) Etats contractants désignés : **AT BE CH DE DK ES FR GB GR IE IT LI LU NL PT SE**

(71) Demandeur : **RHONE-POULENC CHIMIE**
**25, Quai Paul Doumer**
**F-92408 Courbevoie Cédex (FR)**

(72) Inventeur : **Bernard, Jean-Marie**
**Route du Large,**
**Saint-Laurent d'Agny**
**F-69440 Mornant (FR)**

(74) Mandataire : **Ricalens, François et al**
**RHONE-POULENC CHIMIE,**
**Direction de la Propriéte Industrielle,**
**25, quai Paul Doumer**
**F-92408 Courbevoie Cédex (FR)**

## (54) Polyisocyanates masqués.

(57) La présente invention concerne des polyisocyanates masqués, comprenant au moins deux, de préférence au moins trois fonctions isocyanates, caractérisés par le fait qu'ils comportent au moins deux fonctions issues chacune de la réaction d'une fonction isocyanate libre avec un atome à hydrogène mobile d'un composé présentant au moins un cycle à cinq chaînons et à au moins un hétéroatome, ledit cycle présentant au moins une forme mésomère à caractère aromatique et en ce que l'atome par lequel l'hydrogène mobile est rattaché au cycle est choisi parmi les chalcogènes et les éléments de la colonne VB de la classification périodique des éléments telle que publiée dans le supplément au Bulletin de la Société Chimique de France, janvier 1966, n° 1.

Ces produits bénéficient d'une bonne stabilité de protection des fonctions isocyanates, d'une faible température de dissociation et d'une dureté correcte à l'état polymérisé et/ou réticulé.

Application : peintures, revêtements.

EP 0 661 278 A1

Le domaine de l'invention est celui de la chimie et des applications des fonctions isocyanates et des fonctions qui en dérivent, fonctions utiles pour les agents de polymérisation et/ou réticulation.

La présente invention a en particulier pour objet des unités mono-, oligo- ou poly-mères porteuses de groupements isocyanates et réactives avec d'autres co-mono, oligo ou polymères de formule $R^aOH$ et/ou $R^bNH_2$. Plus précisément, la présente invention concerne des polyisocyanates dont certains au moins des groupements fonctionnels sont masqués, ou protégés, par des radicaux $B^{1 \text{ à } n}$, radicaux qui seront parfois identifiés dans la suite de la description par le qualificatif de "bloquant".

La présente invention concerne également certains des procédés d'obtention de ces nouveaux polyisocyanates masqués.
Elle vise en outre l'utilisation des polyisocyanates masqués ci-dessus dans des compositions pour la préparation de polymères, notamment de polycondensats et de réticulats issus de la réaction desdits polyisocyanates protégés et de coréactifs nucléophiles. Cette préparation est celle qui est exploitée dans les applications industrielles, telles que les revêtements en tout genre et notamment ceux sur les textiles, sur les verres, sur les papiers, sur les métaux et sur les matériaux de construction, et les peintures.
L'utilité du masquage des fonctions isocyanates (masquage désigné parfois par blocage), voire sa nécessité, s'explique par une réactivité trop élevée à température ambiante, du comonomère isocyanates vis à vis de l'autre coréactif $R^aOH$ ou $R^bNH_2$ ou vis à vis d'un solvant réactif, ou d'une phase, en général continue, support dans le cas d'émulsions ou de suspensions, tel que l'eau. Cette réactivité élevée est souvent très gênante notamment pour certaines applications des polyuréthannes, en particulier dans les peintures, car cela impose un conditionnement et parfois une manipulation séparés du co-monomère isocyanate. Il en découle une mise en oeuvre peu commode.

Ainsi, dans toutes les applications des polyuréthannes comme revêtements, il est du plus grand intérêt de disposer d'isocyanates protégés, dans lesquels la fonction isocyanate est rendue non réactive à température ambiante vis-à-vis de ses coréactifs, mais maintenue réactive à une température plus élevée.
Ces unités isocyanates masquées sont avantageuses à plusieurs titres. En premier lieu, elles permettent de proposer, dans un seul et même conditionnement, des compositions (y compris émulsions et suspensions) pour l'obtention de revêtement dont le composant isocyanate soit stable et peu sensible à l'eau. Il s'ensuit qu'il n'est plus nécessaire d'utiliser des solvants anhydres onéreux, spécifiques des isocyanates et qu'il est possible de conserver longtemps, sans dégradation, les isocyanates masqués dans des conditions où ceux qui sont libres se dégraderaient.
Enfin, la mise en oeuvre de polyisocyanates masqués permet de réduire, voire d'éliminer, l'éventuel risque toxique associé à la présence d'isocyanates libres et instables.

L'amélioration de cette technique de masquage des groupements fonctionnels isocyanates sur des unités mono-, oligo- ou polymères réactives, passe par l'optimisation, en général un abaissement, de la température de réaction, c'est-à-dire celle à laquelle la déprotection s'effectue, conduisant ainsi à la polymérisation e/ou la réticulation visées.
Plus spécifiquement la température de démasquage doit être suffisamment haute pour qu'il n'y ait pas de risque de réaction pendant la période de stockage et cette température de réaction doit être suffisamment basse pour qu'il soit aisé de réaliser la polycondensation lorsque cela est désiré.
En général la température de "libération" des isocyanates, notamment aliphatique (c'est-à-dire que le carbone porteur de l'azote est d'hybridation sp3) est trop élevée ce qui implique que l'optimisation soit un abaissement de température de réaction.

A titre incident il convient de signaler que les groupes masquants utilisés dans le cas des isocyanates aromatiques ne sont en général pas transposable pour les isocyanates aliphatiques, la température de "libération" pour un même groupe masquant étant de plusieurs dizaines de degrés centigrades supérieure à celle des isocyanates aromatiques.
Un tel abaissement se traduirait, en effet, directement par des gains économiques non négligeables en énergie et en durée de procédé.
Comme cela est indiqué dans le document "SHELBY & BOYER - Journal of Coatings Technology - vol. 62, no. 784, may 1990 - pages 51-63, ce paramètre important qu'est la température de dissociation dépend, en partie, de la nature du groupe masquant.
Toujours selon ce document antérieur, de nombreux radicaux bloquants ont déjà été utilisés. Parmi ceux-ci, on peut citer, entre autres certains, des triazoles, des imidazolines, des lactames, des composés hydroxynitrés, des bisulfites de sodium, des dimères d'isocyanate, des phénols, des esters d'acide acétoacétique et des alcools. Ces radicaux bloquants ont, jusqu'alors, surtout été mis en oeuvre sur des isocyanates comportant, au plus, deux fonctions isocyanates.
Or, le greffage de ces radicaux bloquants sur des composés di-, tri et polyfonctionnels est parfois délicat, en raison de la difficulté qui existe à atteindre toutes les fonctionnalités isocyanates d'une même molécule.

De plus, il convient de noter que les agents bloquants évoqués par SHELBY et BOYER, et en particulier le pentachlorophénol, qu'ils mettent en oeuvre dans leurs essais, sont des produits peu disponibles, onéreux et difficiles à manipuler et à utiliser, en raison de leur caractère toxique, explosif et inflammable.
De surcroît, il est apposer que ces radicaux bloquants connus, qui semblent être les composés les plus performants selon SHELBY et BOYER, ne sont pas réellement satisfaisants au regard de la réaction de polymérisation et/ou réticulation subséquente au déblocage.

En effet, cette dernière ne permet pas d'atteindre des polycondensats, et notamment réticulats qui aient une dureté suffisante pour divers applications, notamment à titre de revêtements (par exemple peinture), et ce nonobstant le fait que ces radicaux bloquants impliquent des températures de "libération" relativement basses, donc acceptables.
Il convient de noter que la multiplicité des paramètres rend difficile la systématisation de certaines familles.

Ainsi, l'un des objectifs essentiels de la présente invention est de fournir de nouveaux polyisocyanates à groupements fonctionnels bloqués et ayant une température de dissociation relativement basse pour une durée limitée et avec un rendement de dissociation compatible avec les techniques de polymérisation.
Un autre objectif de l'invention est de foumir de nouveaux polyisocyanates à groupements fonctionnels bloqués, qui ne soient que peu ou pas toxiques.
Un autre objectif de l'invention est de fournir de nouveaux polyisocyanates à groupements fonctionnels masqués, qui ne soient pas de manipulation et de mise en oeuvre dangereuse et/ou délicate.

Un autre objectif de l'invention est de fournir de nouveaux polyisocyanates à groupements fonctionnels bloqués, qui soient économiques.

Un autre objectif de l'invention est de fournir de nouveaux polyisocyanates à groupements fonctionnels bloqués, donnant accès à des polymères (ou plutôt à de polycondensats), éventuellement réticulés, qui satisfassent au cahier des charges des applications.

Un autre objectif de l'invention est de fournir un procédé d'obtention de tels polyisocyanates bloqués.

Un autre objectif de l'invention est de fournir un procédé de préparation de polymères et/ou réticulats à partir desdits polyisocyanates bloqués.

Un autre but de la présente invention est de fournir des compositions comportant des isocyanates masqués des types précédents.

Un autre but de la présente invention est de fournir des compositions de poudre du type précité.

Un autre but de la présente invention est de fournir des émulsions aqueuses comportant des isocyanates de type ci-dessus.

Un autre but de la présente invention est de fournir des suspensions comportant des isocyanates du type ci-dessus.

Ces objectifs, parmi d'autres, sont atteints par la présente invention qui a pour objet des polyisocyanates à groupements fonctionnels protégés ou masqués, comprenant au moins deux, de préférence au moins trois fonctions isocyanates, et caractérisés par le fait que:

- au moins une, avantageusement au moins deux desdites fonctions isocyanates sont issues de la réaction d'une fonction isocyanate libre avec au moins un composé présentant au moins un cycle à cinq chaînons et à au moins un hétéroatome ledit cycle étant aromatique ou présentant au moins une forme mésomère à caractère aromatique et étant porteur d'un atome lui-même porteur d'un hydrogène mobile,
- et que l'atome par lequel l'hydrogène mobile est rattaché au cycle est exocyclique et choisi parmi les chalcogènes et les éléments de la colonne VB.

Le produit de la réaction est essentiellement le produit d'addition sur la double liaison située entre le carbone et l'azote de la fonction isocyanate, où l'atome porteur de l'hydrogène mobile se lie à l'atome de carbone du carbonyle et l'hydrogène à l'azote.
Ainsi l'invention porte notamment sur l'utilisation des dits composés présentant au moins un cycle à cinq chaînons et à au moins un hétéroatome pour masquer tout ou partie des fonctions isocyanates d'un polyisocyanate, c'est à dire un composé présentant plusieurs fonctions isocyanates. Les fonctions non masquées selon la présente invention, peuvent être soit libres, soit masquées par d'autre groupes masquants. Les groupes masquants selon la présente invention sont particulièrement bien adaptés à la protection de ceux des groupements isocyanates qui sont aliphatiques et parmi ceux-là, ceux dont le carbone porteur de l'azote de la fonction isocyanate est saturé ($sp^3$) et porteur d'un hydrogène, de préférence de deux hydrogènes.

Pour s'assurer, une bonne durée de vie au stockage, il est préférable de choisir des fonctions isocyanates masquées dont le test à l'octanol montre une "libération" à 80°C, avantageusement à 90°C, au plus égale à 90 %.

Pour les utilisations en solvant organique en suspension ou en émulsion, il est souhaitable de choisir des fonctions isocyanates masquées dont le test à l'octanol montre une "libération" à 150°C, avantageusement à 140°C, de préférence 130°C, au moins égale à 90 %.

La classification périodique des éléments utilisée dans la présente demande est celle du supplément au Bulletin de la Société Chimique de France, janvier 1966, n° 1.

Les éventuels substituants dudit cycle à cinq chaînons (ou pentacycle), peuvent être variés et être choisis parmi les substituant dont la liste est établie ci-après, avec la condition que les substituants et notamment les fonctions électroattractrices ne comportent pas d'hydrogène aussi ou plus acide que ledit hydrogène mobile

Ledit composé présentant au moins un cycle à cinq chaînons et à au moins un hétéroatome, est avantageusement léger, c'est à dire qu'il contient avantageusement au plus 50 atomes de carbone, de préférence au plus 25, et plus généralement 15 atomes de carbone.

Il peut arriver qu'il y ait sur ledit cycle des positions porteuses d'hydrogène mobile de réactivité similaire à celle de l'hétéroatome exocyclique porteur d'hydrogène. Dans ce cas il est préférable de bloquer la position en substituant l'hydrogène par un radical hydrocarboné du type $R^1$ à $R^3$ avantageusement de C1 à C20, de préférence de C1 à C11 plus préférentiellement de C1 à C7. Comme position qu'il est opportun de bloquer il convient de citer les hétéroatomes du cycle portant un hydrogène dès lors qu'une migration de cet hydrogène n'est pas engagée dans l'écriture de l'équilibre mettant en évidence l'éventuelle forme mésomère aromatique .

Conformément à une disposition préférée de l'invention, le composé à l'hydrogène mobile est choisi parmi les groupements suivants: imides N-hydroxylés tels que succinimides, malimides et maléimides, et substitués ou non, ainsi que ceux de formule suivante:

(I) 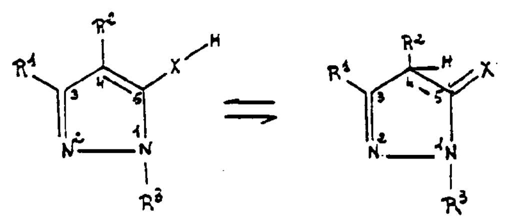 (I')

Ainsi que ceux correspondant à la formule ci-dessus où il y a interversion des chaînons cycliques 1 et 5 et/ou remplacement de N - $R_3$ par un chalcogène; avec:

* X = $NR^{16}$, avantageusement chalcogène, de préférence Soufre ou surtout Oxygène, avec $R^{16}$ choisi parmi l'hydrogène et les restes hydrocarbonés (lesquels ne présentant avantageusement pas de ramification en alpha bêta et gamma de l'azote), de préférence d'au plus 10, avantageusement d'au plus 6 et, plus préférentiellement encore, d'au plus 4 atomes de carbone, le reste hydrocarboné pouvant être acyle (surtout formyle, avantageusement acétyle), alcoyle, aralcoyle, aryle, éventuellement substitué, avec:
* $R^1$, $R^2$ et $R^3$, identiques ou différents, choisi parmi:
  . l'hydrogène;
  . si le chaînon de rattachement du pentacycle n'est pas hétéroatomique, (par exemple cas de $R^1$ et/ou $R^2$) les halogènes avantageusement léger (fluor et chlore), de préférence fluor;
  . des fonctions électroattractrices, tel que carbonyles, y compris carboxylates et amides, nitrile, sulfones, y compris sulfonates, phosphones (c'est-à-dire: -PO=), y compris phosphonates et phosphinates, voire nitro (mais le groupe nitro est souvent dangereux et trop électro attracteur);
  . des radicaux hydrocarbonés, éventuellement substitués, tels que notamment aryles, alcoyles (y compris aralcoyles, cycloalcoyles, hétérocycloalcoyles), et si le chaînon de rattachement du pentacycle n'est pas hétéroatomique, (par exemple cas de $R^1$ et/ou $R^2$), lorsqu'ils sont suffisamment stables, les alcoxyles et acyloxyles;
* deux radicaux vicinaux parmi $R^1$, $R^2$ et $R^3$ pouvant, éventuellement, former un même cycle (y compris hétérocyclique et/ou aromatique) , notamment de formules suivantes:

(II) 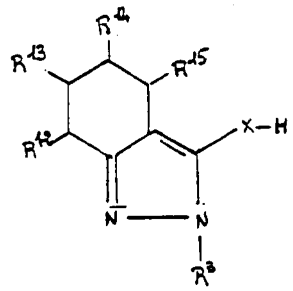

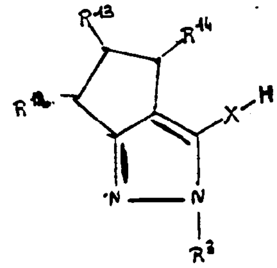 (II')

$R^{12}$ à $R^{15}$ étant identiques ou différents et représentant soit le néant [si le chaînon ne peut porter aucun substituant (c'est le cas par exemple des chalcogènes et/ou des trivalents tel que l'azote dans certaines des positions des cycles aromatiques)] soit des valeurs choisies parmi les mêmes que celles de $R^1$ à $R^3$.

Comme exemple desdites fonctions carbonyles, phosphones ou sulfones on peut citer celles qui répondent respectivement aux formules suivantes:

$$-CO-R^5;$$
$$-P(O)OR'^5-R^5;$$
$$-SO_2-R^5;$$

avec $R^5$ = alcoyle, aryle, aralcoyle, cycloalcoyle, hétérocycloalcoyle, $-NR^6R^7$ ($R^6$ et $R^7$ identiques ou différents pouvant représenter: hydrogène, alcoyle, aryle, aralcoyle, cycloalcoyle, hétérocycloalcoyle);

- O - $R^8$ [$R^8$ étant un radical alcoyle (y compris, aralcoyle, alicyclique et/ou aliphatique, cycloalcoyle, hétérocycloalcoyle ), aryle ou un cation alcalin, tel que Na+ ou K+ forment un sel avec O-, ou correspondant à un groupement alcoyle, aryle, et/ou aryle)];

et avec $R'^5$= alcoyle, aryle, aralcoyle, cycloalcoyle, hétérocycloalcoyle, - $NR^6R^7$ ($R^6$ et $R^7$ identiques ou différents pouvant représenter: hydrogène, alcoyle, aryle, aralcoyle, cycloalcoyle, hétérocycloalcoyle).

Plus précisément, les polyisocyanates masqués selon l'invention présentent des groupements fonctionnels isocyanates protégés par des radicaux bloquants $B^{1\ à\ n}$ et répondent à la formule suivante:

**(III)** $B^1-CO-NH-A-NH-CO-B^2$

dans laquelle A est un squelette qui comprend au moins un groupement $(NH-CO-B^i)_{n-2}$, avec:

- i prenant toutes les valeurs de 3 à n,
- et n = nombre de fonctions isocyanates protégées.

et correspond à : aryle(s), aralcoyle(s), cycloalcoyle(s), hétérocycloalcoyle(s), lesdits radicaux pouvant, éventuellement, être substitués ou non et/ou branchés et/ou azotés et/ou oxygénés et/ou soufrés,

- $B^1$, $B^2$ et $B^i$ étant identiques ou différents, l'un des $B^i$ - CO - NH pouvant éventuellement disparaître au profit d'un groupement terminal NCO et avantageusement trois, de préférence tous, et au moins deux d'entre eux représentant chacun un radical comportant au moins un cycle à cinq chaînons et à au moins un hétéroatome, $B^1$ et $B^2$ étant, de préférence, choisis parmi les groupements issus des composés suivants: succinimide ou maléimide N-hydroxylés et substitués ou non, ceux de formule I ci-dessus.

Ces polyisocyanates ont pour particularité d'avoir au moins deux groupements fonctionnels NCO bloqués par des radicaux $B^1$ et $B^2$, constitués par des radicaux dérivant desdits composés présentant au moins un cycle à cinq chaînons incluant au moins un hétéroatome, de préférence un atome d'azote.

Le choix des radicaux $R^1$ à $R^3$, peut être amené par les considérations ci-après. Si on désire des groupes masquants facilement "libérables" (ou déplaçables) (c'est-à-dire que la température à laquelle la réaction de condensation s'effectue se situe dans la partie basse de la fourchette) , une solution satisfaisante consiste à choisir comme chaînons intracycliques deux hétéroatomes.

De même un électroattracteur (tel que aryle ou acyle) sur ledit cycle à cinq chaînons et notamment sur un hétéroatome intracyclique permet d'abaisser la température de "libération" (ou de déplacement) par rapport au cycle non substitué ou substitué par des groupes donneurs tels que les groupes alcoyles. Et réciproquement, pour élever la température de "libération" (un bon test pour l'évaluation de la température de libération est le test à l'octanol, cf. exemples) on peut diminuer le nombre des hétéroatomes intracycliques et/ou remplacer les groupes électroattracteurs par des groupes électrodonneurs ou par des hydrogènes.

Un des mérites de la Demanderesse est d'avoir sélectionné ce groupe spécifique d'agents bloquants particulièrement efficaces vis-à-vis d'unités polyfonctionnelles réactives, qui peuvent être de nature mono, oligo ou polymère. Ces nouveaux groupements bloquants satisfont aux objectifs évoqués ci-avant et sont, en outre, parfaitement neutres vis-à-vis des transformations ultérieures en polymères.

De plus, les polyisocyanates selon l'invention sont aisés à obtenir. En particulier, ils ne donnent pas lieu, malgré leur nature polyisocyanique, à un greffage incomplet indésiré en radicaux bloquants sur une même molécule.

Enfin, les mélanges bi- ou poly-composants, comportant au moins les polyisocyanates bloqués selon l'invention et les réactifs correspondants ($R^aOH$ et/ou ceux des $R^bNH_2$ qui ne catalyserons pas la "libération" et/ou d'éventuels catalyseurs avantageusement latents) sont parfaitement stables à température ambiante pendant de longues durées.

Il est à noter que de tels mélanges font également partie de l'invention.

Selon une disposition avantageuse de l'invention, les radicaux bloquants $B^i$ peuvent être de nature différente pour un même polyisocyanate. Il convient de souligner l'originalité de cette classe de polyisocyanates masqués

de façon hétérogène, classe qui rentre également dans le cadre de l'invention.

Les polyisocyanates préférés visés par l'invention sont ceux dans lesquels au moins une, avantageusement deux, de préférence trois des conditions ci après sont remplies :

- au moins une, avantageusement deux, des fonctions NCO à protéger sont reliées à un squelette hydrocarboné, par l'intermédiaire d'un carbone saturé ($sp^3$).
- au moins un, avantageusement deux, desdits carbones saturés ($sp^3$) est porteur d'au moins un, avantageusement deux, hydrogène(s),(en d'autre terme il a été trouvé que l'on obtenait de meilleurs résultats lorsque le carbone porteur de la fonction isocyanate était porteur d'un hydrogène, de préférence de deux hydrogènes;
- tous les carbones par l'intermédiaire desquels les fonctions isocyanates sont reliées au squelette hydrocarboné, sont des carbones saturés ($sp^3$), lesquels sont avantageusement en partie, de préférence en totalité, porteurs d'un hydrogène, de préférence de deux hydrogènes;
- sont en particulier bien adaptés ceux qui présentent au moins en partie un squelette isocyanurique ou biuret (que ce squelette soit issus d'un seul ou de plusieurs monomères, voir ci-dessous).

Lorsque les polyisocyanate sont relativement lourds c'est à dire qu'ils comportent au moins 4 fonctions isocyanates masquées, les deux premières conditions deviennent:

- au moins un tiers, avantageusement deux tiers, des fonctions NCO à protéger sont reliées à un squelette hydrocarboné, par l'intermédiaire d'un carbone saturé ($sp^3$).
- au moins un tiers, avantageusement deux tiers, desdits carbones saturés ($sp^3$) est porteur d'au moins un, avantageusement deux, hydrogène(s),(en d'autre terme il a été trouvé que l'on obtenait de meilleurs résultats lorsque le carbone porteur de la fonction isocyanate était porteur d'un hydrogène, de préférence de deux hydrogènes.

La nature du radical A de la formule (III) est déterminant quant à la définition des diverses sous-familles de produits polyisocyaniques auxquels s'applique l'invention. Ces radicaux A peuvent être issus aussi bien de condensation avec un seul type de monomère (en général diisocyanate) que plusieurs types de monomères. Il est préférable qu'il y ait au moins un monomère dont au moins une des fonctions isocyanates soit aliphatique [carbone porteur de la fonction isocyanate d'hybridation ($sp^3$)et avantageusement porteur d'au moins un, de préférence deux, hydrogène(s)]. Ce sont ces fonctions aliphatique qu'il est préférable de masquer par les techniques de l'invention, les autres fonctions isocyanate pouvant être masquées par des technique déjà connues en elles-mêmes.

Une première sous-famille regroupe les produits dans lesquels A est hétérocyclique et est formé de m chaînons:

$$A = [-N(R^{17})-CO-]_m$$

les radicaux $R^{17}$ étant identiques ou différents entre eux et étant choisis parmi les radicaux suivants:

- $R^{18}$-NH-CO-$B^i$,
- $R^{18}$ -N($P^1$)($P^2$)

avec $R^{18}$ = alcoylène, aralcoylène, alcénylène, aralcénylène et $P^1$ et/ou $P^2$ constituent un lien avec le reste du polyisocyanate.

- un radical hydrogène, [dans ce cas l'(ou les) autre(s) $R^{17}$ est(sont) H], aryle, alcoyle, dont notamment alcoyle de nature polymère du type polyacrylate, polyméthacrylate, polyoléfinique ou de nature polyol, aralcoyle, cycloalcoyle, alcoylcarboxyle, amine,

$R^{17}$ pouvant, éventuellement, comprendre au moins un groupement NCO au sein de la chaîne principale, ou bien encore en position latérale ou terminale par rapport à celle-ci, m étant compris entre 2 et 20, de préférence entre 2 et 10 et, plus préférentiellement encore, entre 2 et 5.

De manière privilégiée, on retient dans cette première sous-famille les produits dans lesquels m = 3 et répondant à la formule qui suit:

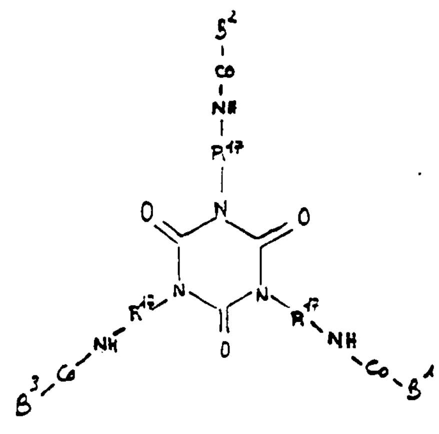

avec $R^{17}$ identiques ou différents entre eux.

A titre d'exemples de produits de cette première sous-famille dans lesquelles m = 3, on peut mentionner:

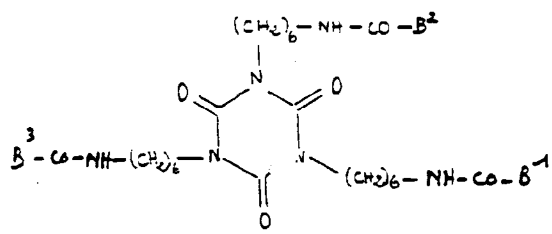

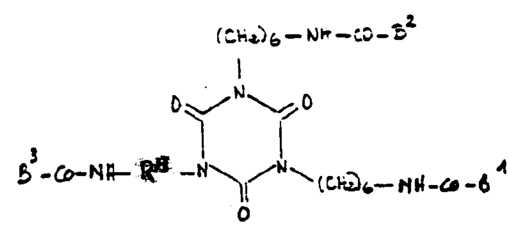

avec $R^{17}$ tel que défini ci-dessus.

Ces produits à squelette isocyanurique sont, bien qu'ils puissent comporter une proportion notable de pentamères, classiquement dénommés "TRIMERES".

Pour compléter l'illustration de cette première sous-famille, on peut citer les produits à squelette diisocyanate, du type :

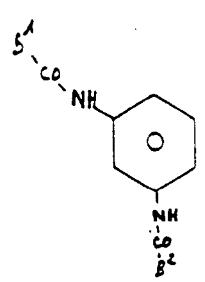

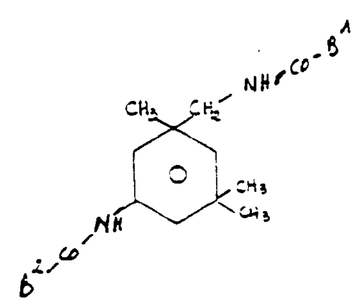

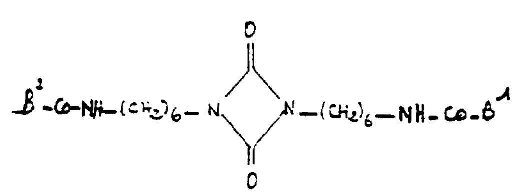

Les polyisocyanates selon l'invention, pour lesquels:

$$A = R^{20} NH -CO -N(R^{19})-CO - NH - R^{21}$$

appartiennent à une deuxième sous-famille selon l'invention.

Dans cette dernière, $R^{19}$, $R^{20}$, $R^{21}$ sont identiques ou différents entre eux et répondent à la formule suivante : - $R^{22}$- NH - CO - $B^i$, avec $R^{22}$ = alcoylène, alcénylène, aralcoylène, aralcénylène.

Ces produits sont classiquement dénommés "BIURET".

A titre d'exemples de radicaux A typiques de cette deuxième sous-famille, on peut mentionner:
$B^1$- CO - NH - $(CH_2)_6$- NH - CO - N[$(CH_2)_6$-NH -CO- $B^2$]- CO - NH - $(CH_2)_6$- NH -CO-$B^3$

Une troisième sous-famille caractéristique de l'invention est celle dans laquelle:

$$A \Rightarrow (A'- L- )_q\mathbf{W}$$

q pouvant être égal à 1 ou indiquer qu'il s'agit d'un oligomère ou d'un pré-polymère, L étant un lien avec au moins une unité mono- ou, L représentant, de préférence $(CH_2)_p$, avec p compris entre 2 et 20, de préférence entre 4 et 10.

A' répondant à la définition de A donnée ci-dessus et L étant un lien avec au moins une unité monomère apte à se (co)polymériser ou une unité polymère, de préférence de nature polyacrylique, polyméthacrylique, polyoléfinique, polyuréthanne, polyester, polyéther, ou avec une unité polymère W, de préférence de nature polyacrylique, polyméthacrylique, polyoléfinique, polyuréthanne, polyester, polyéther.

Selon une modalité intéressante de l'invention, L représente, de préférence, $(CH_2)_p$ avec p compris entre 2 et 20, de préférence entre 4 et 10.

Une quatrième sous-famille vise les isocyanates issus d'une prépolymérisation avec des polyols, en général des triols ou des polyamines, en général des triamines, avec un polyisocyanate, en général diisocyanate, la quantité de fonction isocyanate étant supérieure à celle des fonctions à hydrogène mobile (comme les amines et/ou les alcools), de manière qu'à la fin de la prépolymérisation le nombre de fonctions isocyanates résiduelles par molécule, soit en moyenne supérieur à deux, avantageusement au moins égal à 2,5, de préférence au moins égale à trois.

Conformément à une modalité avantageuse de rinvention, les polyisocyanates dont les NCO sont destinés à être protégés par les radicaux bloquants $B^{1 \text{ à } n}$, sont choisis parmi les produits d'homo- ou d'hétéro-condensation d'alcoylènediisocyanate, avantageusement hexaméthylène diisocyanate, comprenant notamment des produits du type "BIURET" et du type "TRIMERES", et parmi les mélanges en contenant.

Il peut s'agir, par exemple, des polyisocyanates commercialisés par la Société demanderesse sous la dénomination "TOLONATE".

Comme cela ressort de ce qui précède, l'invention trouve également l'un de ses fondements dans le choix des radicaux bloquants $B^{1 \text{ à } n}$, de formule (I) et (I') ou correspondant au succinimide ou au maléimide N-hydroxylés et substitués ou non.

Avec les composés (II) et (II'), le greffage de "B" sur le squelette polyisocyanate est réalisé par l'intermédiaire du substituant X porteur de l'hydrogène mobile.

S'agissant des hydroxy-imides tels que les hydroxysuccinimides et des hydroxymaléimides, leur ancrage doit normalement s'opérer par l'intermédiaire de l'oxygène du N-hydroxyle support de l'hydrogène mobile, avec formation d'une liaison ester et transfert d'un hydrogène à l'azote de la fonction isocyanate.

En général il est préférable que les radicaux bloquants $B^1$ à $B^n$ saturent toutes les fonctions isocyanates non déjà protégées du polyisocyanate à protéger, mais il n'est pas exclu que l'une ou plusieurs de ces fonctions isocyanates restent à l'état libre. C'est généralement le cas pour les produits intermédiaires lorsque l'on veut obtenir des composés masqués par différents groupes masquants.

Concernant les substituants $R^1$, $R^2$, $R^3$, des radicaux bloquants (I) et (I'), on préfère sélectionner $R^1$ et $R^2$:

- soit parmi les alcoyles inférieurs de $C_1$ à $C_6$, comme par exemple les radicaux méthyle, éthyle, propyle, N-butyle, tertiobutyle, pentyle;
- soit parmi les halogénoalcoyles, le ou les halogènes étant chlore ou de préférence fluor ( par exemple : $-CH_2$ - Cl; - $CF_3$) ;
- $CH_2$ - Z, avec Z correspondant à un hétérocycle azoté;
- soit parmi les carboxylates et ou sulfonates alcalin ou d'alcoyle de formule :

  -CO-O-alcoyle;
- soit parmi les cétoalcoyles de formule :

  -CO-alcoyle;
- soit parmi les phényles éventuellement substitués par des radicaux du type électroattracteurs CN, Cl, F, voire $NO_2$;
- soit parmi les acrylates et leurs dérivés, substitués ou non, tels que le méthacrylate.
- les benzoyles, les alcoyles inférieurs de $C_1$ à $C_6$ (méthyle, éthyle, propyle, butyle, tertiobutyle, pentyle ...), substitués ou non, par exemple par les alcoxyles, tels que le méthoxyle, l'éthoxyle.

Pour $R^3$, les phényles, les alcoyles inférieurs de $C_1$ à $C_6$, substitués ou non, de préférence par des halogènes, tels que le brome, le chlore, avantageusement le fluor, les radicaux cyano, font partie des radicaux plus préférentiellement choisis.

Selon une variante $R^1$ et $R^2$ peuvent former un même cycle rattaché au cycle à cinq chaînons des radicaux bloquants $B^1$ à $B^n$ selon l'invention, pour former des composés de formules (II) et (II') mentionnées supra.

Selon une modalité avantageuse de l'invention, il a été procédé à une sélection de radicaux bloquants $B^{1\text{ à }n}$ qui se sont avérés particulièrement, mais non limitativement, appropriés comme constituant structural des nouveaux polyisocyanates ici considérés.

Ces radicaux bloquants préférés sont à structure pyrazole et sont les suivants (nomenclature américaine):
1-phényl-3 méthyl-4 benzoyle pyrazoline-5-one,
3-carboxylate d'éthyle -5-oxo-2-pyrazoline,
3-carboxylate d'éthyle-1-phényl-5-oxo-2-pyrazoline,
1 phényl-3-trifluorométhyl-4,4 diméthyl-5-oxopyrazoline,
1-phényl-3-tertiobutyl-5 oxopyrazoline,
1-méthyl-3 tertiobutyl-5 oxopyrazoline,
p-(3-méthyl-5-oxo-2-pyralin-1-yl) benzoate alcalin,
1 -(3-chlorophényl)-3-tertiobutyl-4-méthylpyrazoline-5-one,
acide-4-[méthyl-5-oxo-2-pyrazoline-1-yl] benzène sulfonate (avantageusement sel de sodium),
3-[3-nitrophényl]-1-phényl-1-pyrazoline-5-one,
3-carboxylate d'éthyle-5-oxo-1-phényl-2-pyrazoline,
4-méthyl-2-pyrazoline-5-one,
1 -phényl-3-trifluorométhyl-2-pyrazoline-5-one,
3-tertiobutyl-2-pyrazoline-5-one,
1-méthyl-3-tertiobutyl-2-pyrazoline-5-one,
3-méthyl-2-pyrazoline-5-one,
1-(3 cyano phényl)-2-pyrazoline-5-one,
1-(3 chlorophényl)-3-méthacrylamide-2 pyrazoline-5 one,
3-méthyl-1-(4 nitrophényl)-2-pyrazoline-5-one,
1,4 dihydro-3 méthyl-4-nitro-1-(4-nitrophényl)-pyrazol-5-one,
4,6-diméthyl, 3-oxopyrazole-3,4-pyridine,
3-méthyl-1-(4-tolyl)-pyrazoline-5-one,
1-(2,5 dichlorophényl)-3 méthyl-5-pyrazolone,
1-(2 chlorophényl)-3-méthyl-5-pyrazolone,
1-(4 chlorophényl)-3-méthyl-5-pyrazolone,
1-(3 nitrophényl)-3-méthyl-5-pyrazolone,
1 -méthyl-3-trifluorométhyl-2-pyrazoline-5-one.

Ainsi, $R^3$ peut être constitué par un noyau aromatique susceptible de comprendre jusqu'à cinq substituants aux hydrogènes benzéniques, lesdits substituants étant identiques ou différents entre eux et sélectionnés, avantageusement, parmi : les halogènes, les radicaux nitro, cyano, alcoyle, aralcoyle, halogéno-alcoyle, hétérocycloalcoyle, carboxyle.

Selon un mode avantageux et particulier de réalisation de l'invention, le composé à hydrogène mobile et apte à réagir, par l'intermédiaire de ce dernier, avec les fonctions isocyanates de manière à les inactiver temporairement, est le N-hydroxysuccinimide.

Et dès lors que les polyisocyanates à protéger sont du type de ceux résultant de la condensation d'hexaméthylène diisocyanate décrits supra (TOLONATE®), on obtient des produits masqués dont l'un des avantages essentiels, outre ceux propres aux polyisocyanates masqués de l'invention, est de se présenter sous forme solide, avantageusement pulvérulente.

Ces polyisocyanates spécifiques (TOLONATE®) protégés à l'aide de N-hydroxysuccinimide constituent un autre objet de l'invention. Ils sont particulièrement intéressants pour des applications de revêtement (par exemple peintures et vernis) en poudre.

Selon un autre aspect, l'invention concerne un procédé d'obtention, notamment de nouveaux polyisocyanates à groupements fonctionnels protégés, tels que ceux définis ci-dessus.

Selon ce procédé, on fait réagir un polyisocyanate de formule OCN-A-NCO, avec A=radical aryle, aralcoyle, cycloalcoyle, hétérocycloalcoyle, le radical pouvant éventuellement être substitué ou non et/ou branché et/ou azoté et/ou oxygéné et/ou soufré et comprendre au moins un groupement amide et/ou un groupement isocyanate, avec au moins un composé à hydrogène ou un radical bloquant $B^{1\text{ à }n}$, tels que définis ci-dessus.

Il s'agit donc d'un polyisocyanate comportant au moins deux groupements fonctionnels NCO, séparés par une chaîne hydrocarbonée différente d'un alcoylène linéaire.

Cette mise en réaction s'opère dans un milieu réactionnel comprenant, avantageusement, du solvant et à une température comprise entre 50 et 95° C, de préférence entre 60 et 70° C et, plus préférentiellement encore, à une température de l'ordre de 65° C.

Concernant la mise en température, il est parfaitement à la portée de l'homme du métier de l'adapter aux produits mis en jeu dans la réaction et, en particulier, d'adopter une procédure de chauffage par paliers progressifs.

Le solvant mis en oeuvre dans ce procédé est un solvant organique.

L'un des nombreux intérêts des nouveaux polyisocyanates selon l'invention est qu'ils peuvent servir de base à la préparation de polymères et/ou de réticulats utiles, par exemple, comme constituants principaux de revêtements en tous genres, tels que des peintures. Dans de telles utilisations, les qualités de dureté des polymères réticulables font partie de celles qui sont recherchées sur le plan technique et fonctionnelles.

La susdite préparation de polymères consiste :

- à mettre les polyisocyanates protégés conformes à l'invention (I) en présence d'un réactif de formule $R^aOH$ et/ou $R^bNH_2$ avec $R^a$ et $R^b$ représentant des groupes hydrocarbonés aliphatiques, alicycliques ou aromatiques, de préférence alcoyles, cycloalcoyles, aryles, aralcoyles, alcényles, aralcényles, linéaires ou branchés, substitués ou non;
- et à chauffer le milieu réactionnel ainsi constitué à une température inférieure ou égale à 150°C, de préférence comprise entre 80 et 140°C et, plus préférentiellement encore, entre 110 et 130° C et ce, pour une durée inférieure ou égale à 15 h, de préférence à 10 h et, plus préférentiellement encore, à 8 h.

Pour satisfaire aux impératifs industriels d'économie et de rentabilité, il est important de prendre en considération ce paramètre temps réactionnel en approchant au mieux le juste compromis température/temps.

Avantageusement, on prévoit d'inclure un solvant organique dans le milieu réactionnel. Ce solvant est, de préférence, non polaire et de constante diélectrique de préférence supérieure ou égale à 4 et, plus préférentiellement encore, à 5.

Conformément à l'invention, les solvants non polaires préférés sont, entre autres, le benzène, le chlorobenzène (1,2-dichlorobenzène), le nitrobenzène, le cyclohexanone, la méthyléthylcétone et l'acétone.

S'agissant du coréactif, en général polyfonctionnel, $R^aOH$ ou $R^bNH_2$ qui peuvent être mono-, oligo- ou polymérique mis en oeuvre pour la préparation de polyuréthannes éventuellement réticulés, son choix sera dicté par les fonctionnalités attendues pour le polymère dans l'application finale. Ces coréactif sont en général bien connu de l'homme de métier.

Selon l'invention et dans la perspective d'élaboration de peintures thermodurcissables, par exemple pour l'automobile, $R^a$ et $R^b$ peuvent être, par exemple, $CH_3 - (CH_2)_7-$.

Ces coréactifs sont naturellement mono- ou polyfonctionnels et peuvent ainsi donner accès à des squelettes polymères variés.

L'invention concerne donc, également, des compositions de peintures comprenant:

- un polyisocyanate masqué selon l'invention;
- un réactif ($R^aOH$ et/ou $R^bNH_2$ et d'éventuels catalyseurs), tels que décrits supra;
- d'éventuels catalyseurs;
- éventuellement au moins un pigment;
- éventuellement une phase aqueuse;
- éventuellement un agent tensio-actif pour maintenir en émulsion ou en suspension les composants constitutifs du mélange;
- éventuellement un solvant organique;

L'invention concerne aussi les peintures et vernis obtenues par l'utilisation de ces compositions, avec l'eventuelle libération selon le procédé ci-dessus.

## APPLICATION AUX COMPOSITIONS A BASE DE POUDRE

Une mention particulière doit être faite pour les hydroxyimides, avantageusement pentacyclique, en effet leur caractère aromatique est très faible et contrairement aux autres membres de la famille des agents bloquants selon l'invention, ils présente une température de "libération" relativement élevée (de 120-130°C) environ (test à l'alcool primaire en général octanol). On considère que la réaction est complète si elle est réalisée à plus de 90 %.).

Toutefois ces imides permettent de résoudre le problème spécifique exposé ci après. Les techniques de revêtement au moyen de poudres se développent de plus en plus. Les isocyanates masqués commencent à y être utilisés mais leur emploi est limité par le peu de composés répondant aux exigences de la chimie des poudres.

Une première difficulté réside dans la difficulté à trouver des isocyanates ou mélanges d'isocyanates masqués qui restent sous forme de poudre dans les conditions de stockage usuelles, conditions qui peuvent varier beaucoup d'un endroit à un autre. Il faut également que ces composés aient des points de transition vitreuse et des points de fusion suffisamment élevés pour qu'ils puissent réagir dans les conditions d'utilisation des

poudres.

En outre Il est préférable que les composés issus des réactions de condensation ne soient délétères ni pour la santé humaine ou animale, ni pour l'environnement.

Il est préférable de choisir les composés de manière qu'ils réagissent complètement avec un alcool primaire à 250°C en moins d'une demi-heure.

C'est pourquoi un but supplémentaire de la présente invention est de fournir une sous-famille d'isocyanates bloqués qui réponde aux contraintes évoquées ci-dessus et qui soit utilisable dans des compositions de poudres pour le revêtement .

Un autre but de la présente invention est de fournir un procédé de synthèse des isocyanates répondant aux contraintes ci-dessus.

Selon la présente invention, on a trouvé qu'une solution résidait dans l'utilisation de d'agent bloquant selon l'invention ou d'hydroxy imide.

Les polyisocyanates préférés visés par rinvention sont ceux dans lesquels au moins une, avantageusement deux, de préférence trois des conditions ci après sont remplies :

- au moins une, avantageusement deux, des fonctions NCO à protéger sont reliées à un squelette hydrocarboné, par l'intermédiaire d'un carbone saturé ($sp^3$).
- au moins un, avantageusement deux, desdits carbones saturés ($sp^3$) est porteur d'au moins un, avantageusement deux, hydrogène(s),(en d'autre terme il a été trouvé que l'on obtenait de meilleurs résultats lorsque le carbone porteur de la fonction isocyanate était porteur d'un hydrogène, de préférence de deux hydrogènes);
- tous les carbones par l'intermédiaire desquels les fonctions isocyanates sont reliées au squelette hydrocarboné, sont des carbone saturé ($sp^3$), lesquels sont avantageusement en partie, de préférence en totalité, porteurs d'un hydrogène, de préférence de deux hydrogènes;
- sont en particulier bien adaptés ceux qui présentent au moins en partie un squelette isocyanurique ou biuret.

Selon la présente invention, il est préférable de choisir le type d'isocyanate et d'hydroxyimide de manière que le point de fusion du composé ou du mélange de composés obtenu présente un point de fusion au moins égal à 50°C.

Il est également préférable que la température de transition vitreuse soit au moins égale à 40°C.

Les compositions des poudres comportent avantageusement au moins un polyol (au moins diol) ou, dans certains cas, au moins une polyamine. on peut également avoir des composés polyfonctionnels présentant au moins deux fonctions choisies parmi les fonctions amines ou ols (phénols ou de préférence alcools) les composés ci-dessus peuvent présenter en outre d'autre fonctions (par exemple fonction acide telle que carboxylique ou sulfonique) à condition que ces fonctions n'empêchent pas la condensation ou la réticulation.

Ces polyols ou polyamines forment également elles-mêmes des poudres et répondent aux mêmes contraintes de point de fusion et de température de transition vitreuse que celles évoquées précédemment.

Il est préférable que le point de fusion des compositions selon la présente invention présente un point de fusion au moins égal à 50°C, il est même souhaitable que les températures de ramollissement soient telles qu'il n'y ait pas frittage de la poudre à une température d'au moins 50°C.

Il est également préférable que leur température de transition vitreuse soit au moins égale à 40°C.

Avantageusement les compositions de poudres comportent également au moins un catalyseur, en général et de préférence des catalyseurs à base d'étain ou de zinc.

Selon la présente invention, le procédé de synthèse consiste à mettre en contact dans un solvant l'isocyanate libre, ou partiellement libre, avec le composé hydroxyimide.

Lorsque les composés selon l'invention et leur précurseurs sont stables dans les conditions ci-après, on peut réaliser la synthèse sans solvant mais à l'état fondu. Le produit final est alors refroidi, par exemple en fabriquant des écailles, qui peuvent être obtenues par le refroidissement brutal occasionné en versant le mélange réactionnel sur une paroi froide. Les écailles obtenues peuvent être broyées.

Lorsqu'il y a solvant, il est de préférence choisi de manière à être suffisamment polaire pour dissoudre au moins 50, de préférence au moins 100, préférentiellement au moins 200 grammes par litre de l'isocyanate initial.

Une fois la réaction terminée, il convient de précipiter le produit final, soit selon une technique classique de cristallisation, et plus préférentiellement par addition d'un composé précipitant suffisamment peu polaire pour provoquer la précipitation sans qu'il y ait nécessairement cristallisation.

Le composé précipitant est bien évidemment un composé de type volatil, et le plus souvent des composés type mélange d'hydrocarbures légers du type éther de pétrole ou du type hexane ou heptane. On peut égalent utiliser, seuls ou en mélange, les éthers d'alcool léger (c'est à dire les alcools comportant au plus six atomes de carbones de préférence 4).

Généralement, on utilise des composés du type alcane ou alcène dont le nombre de carbones est inférieur

à 20 et supérieur à 4.

Selon la présente invention cette aptitude à former des isocyanates masqués bons pour l'utilisation en poudre est une propriété liée aux hydroxyimides, notamment cycliques (hexa- ou pentacycliques) et ce, quoiqu'à un degré moindre, même lorsqu'ils ne sont pas pentacycliques et/ou n'ont pas de caractère aromatique. On peut ainsi citer l'hydroxy-phtalimide et l'hydroxy-glutarimide. Les substituant et les règles de substitution évoquées plus haut sont applicables ici aussi.

L'invention concerne aussi les peintures et vernis obtenues par l'utilisation de ces compositions, avec l'éventuelle libération selon le procédé ci-dessus.

## Avantages

Il ressort de ce qui précède que les nouveaux polyisocyanates et les nouveaux procédés et peintures y associés procurent de nombreux avantages :

- économie,
- atoxicité,
- facilité de mise en oeuvre,
- faible température de dissociation,
- répartition homogène des radicaux bloquants sur les groupes isocyanates.
- stabilisation à température ambiante d'isocyanates difonctionnels exempts de squelettes alcoyles linéaires et de carbamates polyfonctionnels,
- bonne polymérisation et/ou réticulation subséquemment au déblocage.

L'invention trouve un débouché particulièrement intéressant, notamment, dans le domaine des revêtements et peintures pour automobiles, domaine où il importe de réduire la durée et la température d'application et de réticulation des laques et ce, dans des conditions d'hygiène et de sécurité optimales, avec des duretés finales importantes. L'invention sera mieux comprise, ses variantes, ainsi que d'autres de ses avantages ressortiront bien des exemples qui suivent de préparation de polycarbamates bloqués et de polyuréthannes obtenus après déblocage desdits polycarbamates à température élevée.

## TEST A L'OCTANOL

Dans un tube, type SCHOTT, avec agitation magnétique, on charge environ 5 mmol en équivalent NCO masqué protégé à évaluer.

On ajoute 2,5 à 3 ml de dichloro-1,2 benzène (solvant) et l'équivalent d'octanol-1 (5 mmol, soit 0,61 g).

On chauffe 6 h à 110° C, de façon à débloquer et ainsi rendre réactives les fonctions isocyanates.

On évalue le pourcentage de fonction isocyanate masquée condensée avec l'octanol-1.

## EXEMPLES

### EXEMPLE 1:

BLOCAGE DES FONCTIONS ISOCYANATES D'UN COMONOMERE POLYISOCYANATE

Dans un tricol de 500 ml, équipé de moyens d'agitation mécanique et d'un réfrigérant sous argon, on introduit:

- 100 g (0,53 mol en NCO) d'un triisocyanate de formule:

commercialisé par la Société RHONE POULENC sous la dénomination protégée à titre de marque déposée: "TOLONATE HDT",

- 40 g de solvant, du type de celui commercialisé sous la marque déposée "SOLVESSO".

On ajoute, en 4 h, 92,3 g (0,53 mol) de méthyl-3 phényl-1 pyrazoline-2 one-5.

Les premiers 20 % de pyrazoline sont chargés à température ambiante. Puis on chauffe à 55° C. Après 50 % de charge, on monte la température à 65° C et même à 88° C dans le premier quart d'heure.

En fin de charge, on maintient la température à 88° C pendant 3 h.

Après réaction, on obtient une "pâte" orange. Des analyses RMN et IR révèlent que les trois fonctions isocyanates libres du "TOLONATE HDT" sont liées avec des groupements protecteurs:

= phényle.

**EXEMPLE 2:**

TEST DE OCTANOL-1 SUR LE COMONOMERES A FONCTIONS ISOCYANATES BLOQUEES.

Dans un tube, type SCHOTT, avec agitation magnétique, on charge environ 4,7 mmol en NCO bloqué (comonomère A) protégé obtenu à l'exemple 1.

On ajoute 2,5 à 3 ml de dichloro-1,2 benzène (solvant) et l'équivalent d'octanol-1 (coréactif B) (4,7 mmol, soit 0,61 g).

On chauffe (dans un multifour) 6 h à 110° C, de façon à débloquer et ainsi rendre réactives les fonctions isocyanates.

On concentre au maximum la masse réactionnelle, par mise sous vide partiel à l'aide d'une pompe à eau à environ 60° C.

On caractérise en RMN le polyuréthanne (C) formé ["TOLONATE" (A) débloqué de l'exemple 1 + (B) octanol].

Le tableau I ci-dessous présente les résultats obtenus.

**TABLEAU I**

| ANALYSES RMN (% molaire) | | | Rendement en % (C) |
|---|---|---|---|
| Comonomère (A) bloqué fin | Coréactif (B) fin | Polyuréthanne (C) formé | |
| traces | traces | 45 | 100 |

**EXEMPLE 3**

D'autre résultats obtenus selon le mode opératoire des exemples 1 et 2 sont rassemblés dans le tableau ci après:

| Exemple | Agent masquant | Température du test à l'octanol | Rendement de la libération |
|---|---|---|---|
| 3 | méthyl-3 phényl-1 pyrazoline-2 one-5 (produit de l'exemple 1). | 80°C | 80 % |
| 4 | Diméthylpyrazolone (remplacement du phényle du composé précédent par un méthyle). | 110°C | 75 % |
| 5 | mercapto-2 Thiazoline<br>RN CAS N°96-53-7<br>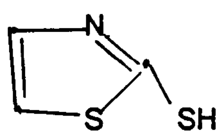 | 110 | Liberation partielle (rdt non mesuré) |
| 6 | mercapto-2 Méthyl-1imidazole<br>RN CAS N° 60-56-0<br>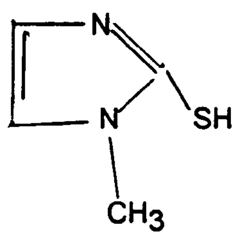 | 110°C | Libération partielle (rdt non mesuré) |
| 7 | Méthyl-4 pyrazoline one-5<br>RN CAS N°13-315-23-6<br>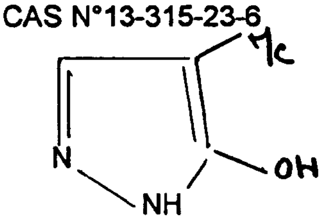 | 110°C | Libération partielle (rdt non mesuré) |
| 8 | Hydroxysuccinimide. | 110°C | Environ 1/3 |

**EXEMPLE 4**

SYNTHESE DU TOLONATE HDT BLOQUE N-HYDROXY SUCCINIMIDE

Dans un réacteur de 500 ml charger:
- Tolonate HDT = 99,7 g (indice NCO = 22 %) (trimère à cycle isocyanurique)
- Chloroforme = 48,1 g

Sous agitation et à température ambiante, ajouter en plusieurs fois.
- Hydroxysuccinimide = 62,1 g (0,54 mole).

La masse réactionnelle est chauffée jusqu'à 60°C et maintenue à cette température jusqu'à disparition

des fonctions NCO.

Après refroidissement, un ajout de n-hexane permet de faire précipiter le polyisocyanate bloqué N-Hydroxysuccinimide.

- n Hexane = 45,3 g

La masse réactionnelle est filtrée, le solide obtenu lavé par plusieurs fractions d'hexane puis broyé et séché à nouveau.

- masse obtenue = 161,8 g
- point de fusion = 90°C

**EXEMPLE 5**

FORMULATION VERNIS DU PRODUIT PRECEDENT

Le produit intermédiaire obtenu (I) est formulé de la façon suivante:

- I = 5,5 g
- Desmophen 690 (*) = 14,5 g (% OH = 2 %)

Soit un rapport NCO/OH = 1

Le mélange des deux poudres est broyé jusqu'à obtenir un mélange parfaitement homogène d'une granulométrie inférieure à 50 μm.

Une fraction de cette poudre est appliquée en couche d'épaisseur 300 μm sur plaque acier et traitée thermiquement à différentes températures pendant 15 minutes.

| T° | 160 | 180 | 200 |
|---|---|---|---|
| Dureté (1) | 350 | >400 | >400 |
| Test solvant (2) | D | D | I |

Le film obtenu est qualifié par la dureté et sa résistance au solvant:

**(1)** Mesurée par pendule de Persoz selon norme NF 30-016

**(2)** Dépôt d'une goutte de MEK (Méthyl - Ethyl Cétone) et observation de la détérioration du fil

D = le film est dégradé par l'action du solvant

I = le film est intact après action du solvant.

**NB**: Un catalyseur type DBTL, Octoate de Zinc ... couramment employé dans la chimie des polyuréthanes peut également être utilisé dans cette application.

**Revendications**

**1 -** Polyisocyanates masqués, comprenant au moins deux, de préférence au moins trois fonctions isocyanates, caractérisés par le fait que:

- au moins une, avantageusement au moins deux desdites fonctions isocyanates sont issues de la réaction d'une fonction isocyanate libre avec au moins un composé présentant au moins un cycle à cinq chaînons et à au moins un hétéroatome ledit cycle étant aromatique ou présentant au moins une forme mésomère à caractère aromatique et étant porteur d'un atome lui-même porteur d'un hydrogène mobile,
- et que l'atome par lequel l'hydrogène mobile est rattaché au cycle est exocyclique et choisi parmi les chalcogènes et les éléments de la colonne VB.

**2 -** Polyisocyanates masqués selon la revendication 1, caractérisés par le fait que le composé à l'hydrogène mobile est choisi parmi les groupements suivants: imides N-hydroxylés tels que succinimides, malimides et maléimides, et substitués ou non, ainsi que ceux de formule suivante:

(I) 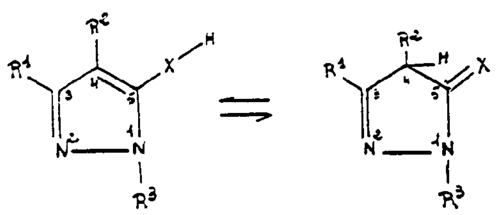 (I')

Ainsi que ceux correspondant à la formule ci-dessus où il y a interversion des chaînons cyclique 1 et 5 et/ou remplacement de N - $R_3$ par un chalcogène avec:

* X = $NR^{16}$, avantageusement chalcogène, de préférence Soufre ou surtout Oxygène, avec $R^{16}$ choisi parmi l'hydrogène et les restes hydrocarbonés de préférence linéaire, avantageusement d'au plus 10, de préférence d'au plus 6 et, plus préférentiellement encore, d'au plus 4 atomes de carbone, le reste hydrocarboné pouvant être acyle , alcoyle, aralcoyle, aryle, éventuellement substitué,
  avec:
* $R^1$, $R^2$ et $R^3$, identiques ou différents, choisi parmi:
  . l'hydrogène;
  . si le chaînon de rattachement du pentacycle n'est pas hétéroatomique, les halogènes avantageusement légers (fluor et chlore), de préférence fluor;
  . des fonctions électroattractrices, tel que carbonyles, y compris carboxylates et amides, nitrile, sulfones, y compris sulfonates, phosphones (c'est-à-dire: -PO=), y compris phosphonates et phosphinates, voire nitro (mais le groupe nitro est souvent dangereux et trop électroattracteur);
  . des radicaux hydrocarbonés, éventuellement substitués, tels que notamment aryles, alcoyles (y compris aralcoyles, cycloalcoyles, hétérocycloalcoyles), et si le chaînon de rattachement du pentacycle n'est pas hétéroatomique, (par exemple cas de $R^1$ et/ou $R^2$), lorsqu'ils sont suffisamment stables, les alcoxyles et acyloxyles;
* deux radicaux vicinaux parmi $R^1$, $R^2$ et $R^3$ pouvant, éventuellement, former un même cycle (y compris hétérocyclique et/ou aromatique).

**3 -** Polyisocyanates masqués selon les revendications 1 et 2, caractérisés en ce qu'ils présentent des groupements fonctionnels isocyanates protégés par des radicaux bloquants $B^{1\ à\ n}$ répondant à la formule suivante:

**(III)** $B^1$ CO - NH - A - NH -CO - $B^2$

dans laquelle A comprend au moins un groupement (NH - CO - $B^i)_{n-2}$, avec:

- prenant toutes les valeurs de 3 à n,
- et n = nombre de fonctions isocyanates protégées,

et correspond à : aryle(s), aralcoyle(s), cycloalcoyle(s), hétérocycloalcoyle(s), lesdits radicaux pouvant, éventuellement, être substitués ou non et/ou branchés et/ou azotés et/ou oxygénés et/ou soufrés,
et répondant à la formule (I) avec ou sans interversion des chaînons cyclique 1 et 5 et/ou remplacement de N - $R_3$ par un chalcogène

- $B^1$, $B^2$ et $B^i$ étant identiques ou différents, l'un des $B^i$ - CO - NH pouvant éventuellement disparaître au profit d'un groupement terminal NCO et avantageusement trois, de préférence tous, et au moins deux d'entre eux représentant chacun un radical, semblable ou différent, issu desdits composés présentant au moins un cycle à cinq chaînons et à au moins un hétéroatome $B^1$ et $B^2$ étant, de préférence, choisis parmi les groupements issus des composés suivants: succinimide ou maléimide N-hydroxylés et substitués ou non, ou de ceux de formule (I) ci dessus avec ou sans interversion des chaînons cycliques 1 et 5 e/ou remplacement de N - $R_3$ par un chalcogène.

**4 -** Polyisocyanates masqués selon la revendication 3, caractérisés en ce que A est hétérocyclique et en ce qu'il est formé de m chaînons:

A = 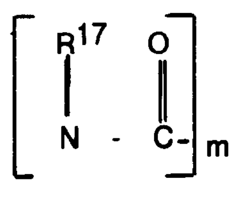

les radicaux $R^{17}$ étant identiques ou différents entre eux et étant choisis parmi les radicaux suivants:
-$R^{18}$ - NH - CO - $B^i$,

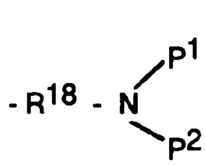

avec $R^{18}$ = alcoylène, aralcoylène, alcénylène, aralcénylène et $P^1$ = ou $P^2$ = définition de $R^{17}$,

- un radical hydrogène, [dans ce cas l'(ou les) autre(s) $R^{17}$ est(sont) H], aryle, alcoyle, dont notamment alcoyle de nature polymère du type polyacrylate, polyméthacrylate, polyoléfinique ou de nature polyol, aralcoyle, cycloalcoyle, alcoylcarboxyle, amine,

$R^{17}$ pouvant, éventuellement, comprendre au moins un groupement NCO au sein de la chaîne principale, ou bien encore en position latérale ou terminale par rapport à celle-ci, m étant compris entre 2 et 20, de préférence entre 2 et 10 et, plus préférentiellement encore, entre 2 et 5.

**5 -** Polyisocyanates masqués selon la revendication 3, caractérisés en ce que:

A =

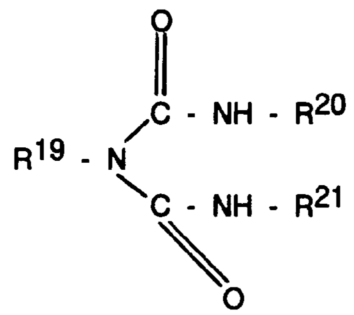

$R^{19}$, $R^{20}$, $R^{21}$ étant identiques ou différents entre eux et répondant à la formule suivante : - $R^{22}$ NH - CO - $B^i$, avec $R^{22}$ = alcoylène, alcénylène, aralcoylène, aralcénylène.

**6 -** Polyisocyanates masqués selon la revendication 3, caractérisés en ce que:

- A - = >A' - L - W

A' répondant à la définition de A donnée dans la revendication 1 et L étant un lien avec au moins une unité mono- ou polymère W, de préférence de nature polyacrylique, polyméthacrylique, polyoléfinique, polyuréthanne, polyester, polyéther, L représentant, de préférence $(CH_2)_p$, avec p compris entre 2 et 20, de préférence entre 4 et 10.

**7 -** Polyisocyanates masqués selon l'une quelconque des revendications 3 à 6, caractérisés en ce qu'ils sont choisis parmi les produits de condensation du type "BIURET" et du type "TRIMERES", comprenant notamment des produits issus de la condensation homogène ou hétérogène d'hexaméthylène diisocyanate.

**8 -** Polyisocyanates masqués selon l'une quelconque des revendications 3 à 6, caractérisés en ce qu'ils sont choisis parmi les produits suivants:

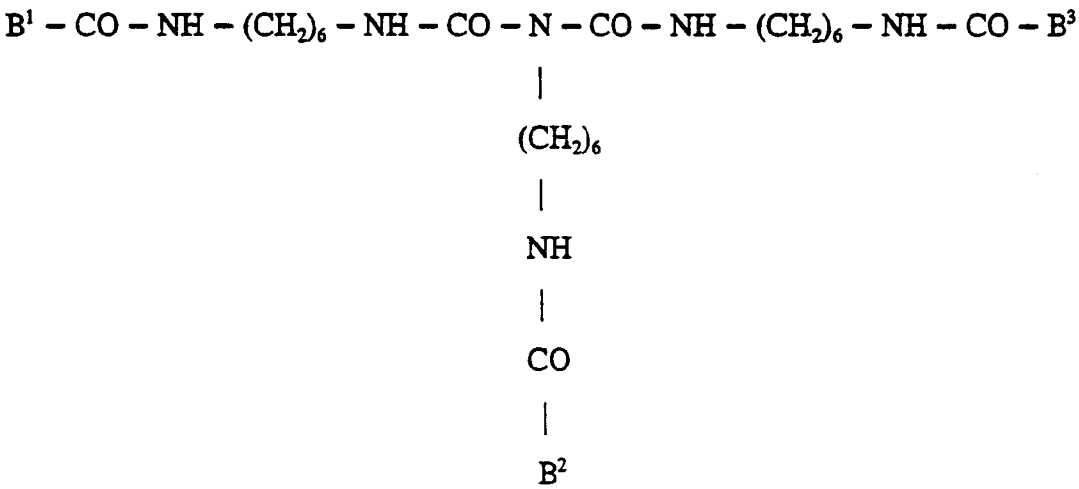

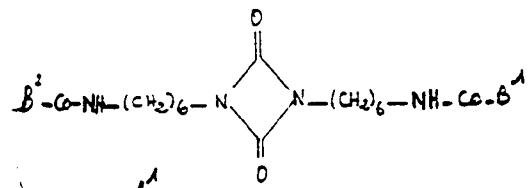

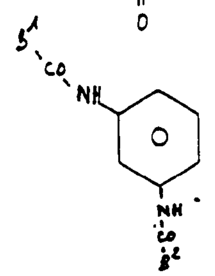

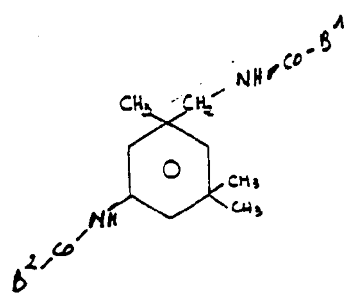

**9** - Polyisocyanates masqués selon l'une quelconque des revendications 3 à 8, caractérisés en ce que $R^1$ et/ou $R^2$ est l'un des substituants suivants:

- soit parmi les alcoyles inférieurs de $C_1$ à $C_6$, comme par exemple les radicaux méthyle, éthyle, propyle, N-butyle, tertiobutyle, pentyle;
- soit parmi les halogénoalcoyles, le ou les halogènes étant chlore ou de préférence fluor ( par exemple : $-CH_2$ - Cl; - $CF_3$);
- $CH_2$ - Z, avec Z correspondant à un hétérocycle azoté;
- soit parmi les carboxylates et ou sulfonates alcalins ou d'alcoyle de formule :

  -CO-O-alcoyle;
- soit parmi les cétoalcoyles de formule:

  CO - alcoyle;
- soit parmi les phényles éventuellement substitués par des radicaux du type électroattracteurs carboxylate et ou sulfonate d'alcalin, CN, Cl, F, voire $NO_2$;
- soit parmi les acrylates et leurs dérivés, substitués ou non, tels que le méthacrylate,
- les benzoyles, les alcoyles inférieurs de $C_1$ à $C_6$ (méthyle, éthyle, propyle, butyle, tertiobutyle, pentyle ...), substitués ou non, par exemple par les alcoxyles, tels que le méthoxyle, l'éthoxyle.

Pour $R^3$, les phényles, les alcoyles inférieurs de $C_1$ à $C_6$, substitués ou non, de préférence par des halogènes, tels que le brome, le chlore, avantageusement le fluor, les radicaux cyano, font partie des radicaux plus préférentiellement choisis.

**10 -** Polyisocyanates masqués selon l'une quelconque des revendications 3 à 9, caractérisés en ce que les radicaux bloquants $B^{1 \text{ à } n}$ sont choisis dans la liste ci-dessous:

1-phényl-3 méthyl-4 benzoyle pyrazoline-5-one,
3-carboxylate d'éthyle -5-oxo-2-pyrazoline,
3-carboxylate d'éthyle-1-phényl-5-oxo-2-pyrazoline,
1-phényl-3-trifluorométhyl-4,4 diméthyl-5-oxopyrazoline,
1-phényl-3-tertiobutyl-5 oxopyrazoline,
1-méthyl-3 tertiobutyl-5 oxopyrazoline,
acide p-(3-méthyl-5-oxo-2-pyralin-1-yl) benzoïque,
4-benzoyl-3-méthyl-1-phényl-2-pyrazoline-5-one,
1-(3-chlorophényl)-3-tertiobutyl-4-méthylpyrazoline-5-one,
4-[méthyl-5-oxo-2-pyrazoline-1-yl] benzène sulfonate (avantageusement sel de sodium),
3-[3-nitrophényl]-1-phényl-1-pyrazoline-5-one,
3-carboxylate d'éthyle-5-oxo-1-phényl-2-pyrazoline,
4-méthyl-2-pyrazoline-5-one,
1-phényl-3-trifluorométhyl-2-pyrazoline-5-one,
3-tertiobutyl-2-pyrazoline-5-one,
1-méthyl-3-tertiobutyl-2-pyrazoline-5-one,
3-méthyl-2-pyrazoline-5-one,
1-(3 cyano phényl)-2-pyrazoline-5-one,
1-(3 chlorophényl)-3-méthacrylamide-2 pyrazoline-5 one,
3-méthyl-1-(4 nitrophényl)-2-pyrazoline-5-one,
1,4 dihydro-3 méthyl-4-nitro-1-(4-nitrophényl)-pyrazol-5-one,
4,6-diméthyl, 3-oxopyrazole-3,4-pyridine,
3-méthyl-1-(4-tolyl)-pyrazoline-5-one,
1-(2,5 dichlorophényl)-3 méthyl-5-pyrazolone,
1-(2 chlorophényl)-3-méthyl-5-pyrazolone,
1-(4 chlorophényl)-3-methyl-5-pyrazolone,
1-(3 nitrophényl)-3-méthyl-5-pyrazolone,
1-méthyl-3-trifluorométhyl-2-pyrazoline-5-one.

**11 -** Polyisocyanates masqués selon la revendication 7, caractérisés en ce que le composé à l'hydrogène mobile est le N-hydroxysuccinimide et en ce qu'ils se présentent sous forme solide.

**12 -** Procédé d'obtention de polyisocyanates à groupements fonctionnels protégés selon l'une quelconque des revendications 1 à 11, caractérisé en ce qu'il consiste à faire réagir un polyisocyanate de formule:

$$OCN - A - NCO$$

avec A = radical aryle, aralcoyle, cycloalcoyle, hétérocycloalcoyle, ledit radical pouvant éventuellement être substitué ou non et/ou branché et/ou azoté et/ou oxygéné et/ou soufré et comprendre au moins un groupement amide et/ou un groupement isocyanate, avec au moins un composé à hydrogène mobile ou un radical bloquant $B^{1 \text{ à } n}$, en présence de solvant et à une température comprise entre 50 et 95° C, de préférence entre 60 et 70° C et, plus préférentiellement encore, de l'ordre de 65° C.

**13 -** Procédé de préparation de polymères, éventuellement réticulés, à partir de polyisocyanates protégés selon l'une quelconque des revendications 1 à 11, caractérisé en ce que:

- on met les polycarbamates protégés (I) en présence d'un comonomère $R^aOH$ et/ou $R^bNH_2$, avec $R^a$, $R^b$ représentant des groupements hydrocarbonés, aliphatiques, alicycliques ou aromatiques, de préférence alcoyle, cycloalcoyle, aryle, aralcoyle, alcényle, aralcényle, linéaires ou branchés, substitués ou non,
- et on chauffe le milieu réactionnel à une température inférieure ou égale à 150°C, de préférence comprise entre 100 et 140°C, plus préférentiellement encore entre 110 et 130° C, pour une durée inférieure ou égale à 15 h, de préférence à 14 h et, plus préférentiellement encore, à 8 h.

**14 -** Procédé selon la revendication 13, caractérisé en ce que l'on prévoit d'inclure un solvant organique dans le milieu réactionnel, ce solvant étant, de préférence, non polaire, de constante diélectrique, de préférence supérieure ou égale à 4 et, plus préférentiellement encore, à 5.

**15 -** Application des polyisocyanates masqués selon l'une quelconque des revendications 1 à 11 à titre de constituants de peintures.

**16 -** Composition de peinture, caractérisée en ce qu'elle comprend:

- un mélange d'au moins un polyisocyanate masqué selon l'une quelconque des revendications 1 à 11 et
- un réactif ($R^aOH$ et/ou $R^bNH_2$ et d'éventuels catalyseurs).

**17 -** Composition selon la revendication 16 caractérisée par le fait que elle est sous forme de poudre.

Office européen des brevets

# RAPPORT DE RECHERCHE EUROPEENNE

Numero de la demande
EP 94 40 2966

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.6) |
|---|---|---|---|
| X | WO-A-86 00630 (THE DOW CHEMICAL COMPANY) * page 6, ligne 27-30; page 7, ligne 28-30 * | 1,2 | C07D251/34 C07D229/00 C08G18/80 |
| X | US-A-5 252 689 (AMERICAN CYANAMID COMPANY) * colonne 2-3 * | 1,2 | |
| X | US-A-4 507 412 (THE DOW CHEMICAL COMPANY) * colonne 3, liggne 56- colonne 4, ligne 10; colonne 4, ligne 20-23 * | 1,2 | |
| X | EP-A-0 193 828 (BASF AG) * page 21, 2me partie: 2-mercaptobenzthiazol * | 1,2 | |
| A | US-A-4 797 455 (W.R. GRACE & CO.) * colonne 1-2 * | 1,2 | |
| A | EP-A-0 500 495 (CIBA-GEIGY AG) * revendications * | 1,2 | |

DOMAINES TECHNIQUES RECHERCHES (Int.Cl.6)

C07D
C08G

Le présent rapport a été établi pour toutes les revendications

| Lieu de la recherche | Date d'achèvement de la recherche | Examinateur |
|---|---|---|
| LA HAYE | 22 Février 1995 | Van Bijlen, H |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)